# EUROPEAN PATENT SPECIFICATION

(11) **EP 3 773 444 B1**
(45) Date of publication and mention of the grant of the patent: **02.03.2022**
(21) Application number: 18725745.6
(22) Date of filing: 05.04.2018
(51) Int. Cl.: A61K 8/37, A61K 8/81, A61Q 15/00, A61Q 17/04

(54) **COSMETIC COMPOSITION FOR A FAST SWEAT EVAPORATION**
KOSMETISCHE ZUSAMMENSETZUNG FÜR EINE SCHNELLE SCHWEISSVERDAMPFUNG
COMPOSITION COSMETIQUE POUR UNE EVAPORATION RAPIDE DE LA SUEUR

(43) Date of publication of application: 17.02.2021
(73) Proprietor: Beiersdorf AG, 20253 Hamburg (DE)
(72) Inventor: DE MORAL, Yanel, León, 37296 (MX); MORAN, Judith, León, 37138 (MX); SCHWANKE, Frank, 22527 Hamburg (DE); CRUZ CERVANTES, Osvaldo José Fabián, León, 37530 (MX)
(74) Representative: Beiersdorf AG
(86) International application number: PCT/EP2018/058660
(87) International publication number: WO 2019/192695

(56) References cited:
- WO-A1-00/47169
- WO-A1-2012/149355

## Description

The present invention belongs to the cosmetic field and relates to the use of polar oils in a cosmetic composition to allow for a quick evaporation of water and/or sweat from the skin, on which the cosmetic composition was applied to.

A beautiful and attractive appearance is a desire for many people. One typical sign of such an appearance is a healthy and smooth looking skin. Therefore, in order to take care on the skin, it is for many people a daily routine to apply cosmetic products such as body lotions, sunscreens or deodorant compositions.

Especially under warm and humid weather conditions it essential for humans to control their body temperature by the production of sweat. Although this is a normal and an essential process to stay healthy, the appearance of visible sweat is in most cultures not very esthetic. This is mainly due to the fact that bacteria present on the skin degrade sweat components which leads to the appearance of an unpleasant odor. Hence, once sweat becomes visible on the skin people expect the person to have an unpleasant odor which may lead to a social isolation.

Generally, the most sweat is released/produced in the axillary region of the body. However, as sweat glands are located all over the body the sweet may also appear in other regions. For people who tend to an increased perspiration it is therefore important to avoid the visible appearance of sweat on the skin.

One way to reduce the appearance of sweat on the skin is to apply cosmetic compositions proving an antiperspirant effect by blocking the sweat glands. Typically such an effect is achieved by the use of aluminum salts in the cosmetic composition. However, in some global regions customers prefer to use cosmetic products which are free from aluminum salts. Furthermore, by applying antiperspirant products all over the body the temperature regulation of the human body may be affect. This effect may not be desirable for most of the people.

Another approach to reduce the appearance of visible sweat on the human body is to apply cosmetic composition containing a water absorbing ingredient. Such ingredient may be polysaccharides such as starch derivatives, e.g. sodium hydroxypropyl starch phosphate. However, such composition usually has the disadvantage that white residues remain on the skin, what is rather unpleasant for the consumer.

For people with dry skin it is an important procedure to apply a body lotion once a day to increase the elasticity and suppleness of the skin. However, the use of such products is often associated with certain disadvantages. For example, it is problematic for people who tend to have an increased perspiration that due to the body lotion the water from sweat remains on the skin for a prolonged period of time. As discussed before this fact is rather annoying for the consumer for esthetic reasons.

Therefore, it is desirable to provide caring body lotion or sunscreen products which do not suffer the disadvantages discussed above. In particular, it is desirable that after application of a body lotion or sunscreen to the skin water and/or sweat can quickly evaporate from the skin. Thereby, it is desirable to avoid the use of antiperspirant actives, in particular the use of aluminum salts. Furthermore, it is desirable that the ingredients contained in the cosmetic composition do not form white residues by the absorption of water, such as described above for the some polysaccharides.

The document US 6319491 B1 discloses an anti-sweat lotion to control the perspiration. However, the document could not direct the person skilled in art to the present invention, as it does not disclose a way to increase the sweat evaporation rate. Instead, it is directed to avoiding perspiration.

It was surprisingly found that the objectives described above can be solved by the present invention.

The invention is the use of an oil a) having a dielectric constant of at least 4.0 in a cosmetic composition to increase after application of the composition to the human skin the evaporation rate of water and/or sweat from the skin.

The term "after application" refers herein to a period of 12 hours, preferably 6 hours starting with the application of the cosmetic composition.

A further object of the disclosure is the use of an oil a) having a dielectric constant of at least 4.0 in a cosmetic composition to reduce the appearance of sweat on the human skin.

Still another object of the disclosure is the use of an oil a) having a dielectric constant of at least 4.0 in a cosmetic composition to reduce the appearance of sweat stains on clothing, worn on the skin after application of cosmetic composition to the skin.

According to the disclosure sweat stains are understood as the wet appearance of clothing from sweat.

A further object of the invention is a method to increase the water and/or sweat evaporation rate on the skin characterized in that a cosmetic composition containing an oil a) having a dielectric constant of at least 4.0 is applied to the skin before the skin becomes wet and/or before perspiration starts.

The cosmetic compositions according to the invention allow for a surprisingly fast evaporation of water and/or sweat from skin preconditioned with the cosmetic composition. Hence, another aspect of the invention is the cosmetic composition as disclosed herein. According to the invention oils generally are understood as water immiscible organic substances, which are liquid at 20°C and 1013 hPa.

In the following description the terms "according to the invention", "preferred according to the invention" and so on are always directed to the use according to the invention, as defined in the appended claims.

All weight percentages (% by weight) given below relate, unless otherwise stated, to the total weight of the cosmetic composition. If ratios of certain components are disclosed in the following description, these ratios refer, unless otherwise stated, to weight ratios of the components.

Unless otherwise stated, all tests and measurements were performed under "normal conditions". The term "normal conditions" refers to 20°C, 1013 hPa and a relative humidity of 50%.

According to the present invention, all values given for the dielectric constant refer to a temperature of 25°C and a pressure of 1013 hPa.

According to the present invention the dielectric constant of an oil can be determined using dielectric constant meter, e.g. BI-870 Dielectric Constant Meter from the Fa. Brookhaven Instruments Corporation, USA.

The term "skin" refers solely to human skin.

In terms of the present invention it is preferred if the oil a) is characterized in that the dielectric constant is at least 4.5, more preferably at least 5.0 and most preferably at least 5.2.

Moreover, it is preferred if the oil a) is characterized in that the dielectric constant is less than 8.0, more preferred less than 7.0 and most preferred less than 6.1.

Following the limitation of the lower and upper limit of the dielectric constants for the oil a) it is also advantageous, if the oil a) is selected from the group of oils having a dielectric constant in the range from 4.5 to 8.0, preferably 5.0 to 7.0 and more preferably 5.2 to 6.1.

Furthermore, it is preferred if the oil a) having a dielectric constant as specified according to invention contains at least one ester group in its molecular structure.

Table 1 indicates a number of oils and their corresponding dielectric constants at 25°C.

**Table 1:**

| INCl | Dielectric constant |
|---|---|
| Isodecyl Malate | 6.0 |
| Lauryl Lactate | 5.6 |
| C-12-15 Alkyl Lactate | 5.4 |
| Diisopropyl Adipate | 5.4 |
| Diisopropyl Sebacate | 4.7 |
| Diethylhexyl Adipate | 4.2 |
| Butyl Oleate | 4.0 |
| Caprylic/Capric Triglycerides | 3.8 |
| C12-15 Alkyl Benzoate | 3.8 |
| Isopropyl Myristate | 3.2 |
| Ethylhexyl Palmitate | 3.1 |
| Ethylhexyl Stearate | 3.1 |
| Isocetyl Stearate | 3.0 |
| Octyldodecyl Stearate | 2.8 |
| Mineral Oil | 2.1 |

Furthermore, it is preferred, if the oil a) having a dielectric constant as specified according to the invention is preferably not an organic UV-filter.

According to the invention organic UV-filters are those organic compounds which are known from the list of UV-filters allowed in cosmetic products from Annex VI of the Regulation (EC) No 1223/2009 of the European Parliament and of the Council, including all amendments and corrections made until the 01.11.2017. The Regulation (EC) No 1223/2009 of the European Parliament and of the Council, the amendments and corrections can be accessed via the website http://eur-lex.europa.eu/homepage.html.

According to the present invention it is preferred if the oil a) is chosen from the group of isodecyl malate, C12-15 alkyl lactate, lauryl lactate, dibutyl adipate, diisopropyl adipate, diethylhexyl adipate and diisopropyl sebacate. It is even more preferred if the oil a) is chosen from the group of isodecyl malate, C12-15 alkyl lactate, lauryl lactate and diisopropyl adipate. It is most preferred if the oil a) is diisopropyl adipate.

Furthermore, it is also preferred if the total quantity of the oil a) as specified above in the cosmetic composition is in the range from 1% to 20% by weight, more preferably from 2% to 10% by weight and most preferably from 3% to 7% by weight, calculated to the total weight of the composition.

It is more preferred, if the total quantity of the oil a) selected from the group of isodecyl malate, C12-15 alkyl lactate, lauryl lactate, dibutyl adipate, diisopropyl adipate, diethylhexyl adipate and diisopropyl sebacate is in the range from 1% to 20% by weight, more preferably from 2% to 10% by weight and most preferably from 3% to 7% by weight, calculated to the total weight of the composition.

It is even more preferred, if the total quantity of the oil a) selected from the group of isodecyl malate, C12-15 alkyl lactate, lauryl lactate and diisopropyl adipate is in the range from 1% to 20% by weight, more preferably from 2% to 10% by weight and most preferably from 3% to 7% by weight, calculated to the total weight of the composition.

According to the invention it is most preferred if diisopropyl adipate is selected as oil a) and the total quantity of diisopropyl adipate is in the range from 1% to 20% by weight, more preferably from 2% to 10% by weight and most preferably from 3% to 7% by weight, calculated to the total weight of the composition.

Furthermore, it is preferred according to the invention if the cosmetic composition contains at least one silicone oil in a total quantity of 0.05% to 2% by weight, more preferably 0.1% to 2% by weight and most preferably 0.2% to 0.5% by weight, calculated to the total weight of the composition. According to the invention it is further preferred if the silicone oil contained in the cosmetic composition of the invention is a non-cyclic polydimethylsiloxane.

According to the present invention it is further preferred, if the cosmetic composition contains all non-silicone oils, which have a dielectric constant of less than 4.0 at 25°C and which are not an organic UV-filter, in a total quantity of less than 1.5% by weight, more preferably less than 0.8% by weight, more preferably less than 0.5% by weight, more preferably less than 0.1% by weight and most preferably 0% by weight, calculated to the total weight of the composition.

It is further especially preferred, if the cosmetic composition contains C12-15 alkyl benzoate, mineral oil, caprylic/capric triglyceride and/or ethylhexyl palmitate in a total quantity of less than 0.2% by weight, more preferably less than 0.1% by weight and most preferably 0% by weight, calculated to the total weight of the composition.

In some embodiments of the invention, it is further preferred if the cosmetic composition comprises the skin conditioning agent butylene glycol dicaprylate/dicaprate. If the composition contains butylene glycol dicaprylate/dicaprate, it is further preferred if the total quantity of butylene glycol dicaprylate/dicaprate is in the range from 0.2% to 1.5% by weight, calculated to the total weight of the composition.

According to the invention, it is further preferred if the cosmetic composition comprises butyrospermum parkii (shea butter) in a total quantity from 0.1% to 1.5% by weight, calculated to the total weight of the composition.

Furthermore, it was surprisingly noticed that the addition of cetyl alcohol and/or stearyl alcohol reduces tackiness of the cosmetic composition during application. The addition of behenyl alcohol did not reduce the tackiness during application. Therefore, it is further preferred, if the cosmetic composition contains cetyl alcohol and/or stearyl alcohol. Mixtures of cetyl alcohol and stearyl alcohol can be obtained under the INCl designation cetearyl alcohol. If the composition contains cetyl alcohol and/or stearyl alcohol, it is further preferred, if the total quantity of cetyl alcohol and/or stearyl alcohol is in the range from 0.2% to 4% by weight, more preferably 1% to 3% by weight, calculated to the total weight of the composition.

Furthermore, preferred embodiments of the present invention are therein characterized that the composition contains at least one organic UV-filter.

Organic UV-filters which are preferred according to the invention are selected from the group of camphor benzalkonium methosulfate, homosalate, benzophenone-3, phenylbenzimidazole sulfonic acid, terephthalylidene dicamphor sulfonic Acid, butyl methoxydibenzoylmethane, benzylidene camphor sulfonic acid, octocrylene, polyacrylamidomethyl benzylidene camphor, PEG-PABA, isoamyl P-methoxycinnamate, ethylhexyl triazone, drometrizole trisiloxane, diethylhexyl butamido triazone, 4-methylbenzylidene camphor, ethylhexyl salicylate, ethylhexyl dimethyl PABA, benzohenone-4, benzophenone-5, methylene bis-benzotriazolyl tetramethylbutylphenol, disodium phenyl dibenzimidazole tetrasulfonate, bis-ethylhexyloxyphenol methoxyphenyl triazine, polysilicone-15, diethylamino hydroxybenzoyl hexyl benzoate and tris-biphenyl triazine. Organic UV-filters which are more preferred are selected from the group of homosalate, phenylbenzimidazole sulfonic acid, butyl methoxydibenzoylmethane, octocrylene, ethylhexyl triazone, ethylhexyl salicylate, benzohenone-4, bis-ethylhexyloxyphenol methoxyphenyl triazine and polysilicone-15. The organic UV-filters which are most preferred according to the invention are selected from the group of bis-ethylhexyloxyphenol methoxyphenyl triazine, homosalate, ethylhexyl salicylate, octocrylene and butyl methoxydibenzoylmethane.

According to the invention it is preferred if the total quantity of the organic UV-filters contained in the cosmetic composition is in the range from 1% by weight to 35% by weight, more preferably 8% by weight to 30% by weight and most preferably 10% by weight to 28% by weight, calculated to the total weight of the composition.

In a preferred case of the invention the cosmetic composition contains at least one organic UV-filter selected from the group of bis-ethylhexyloxyphenol methoxyphenyl triazine, homosalate, ethylhexyl salicylate, octocrylene and butyl methoxydibenzoylmethane, whereby the total quantity of the organic UV-filters selected from the group of bis-ethylhexyloxyphenol methoxyphenyl triazine, homosalate, ethylhexyl salicylate, octocrylene and butyl methoxydibenzoylmethane is in the range from 1% by weight to 35% by weight, more preferably 8% by weight to 30% by weight and most preferably 10% by weight to 28% by weight, calculated to the total weight of the composition.

In the case that the cosmetic composition of the present invention contains bis-ethylhexyloxyphenol methoxyphenyl triazine it is preferred if the total quantity of bis-ethylhexyloxyphenol methoxyphenyl triazine is in the range from 0.1% by weight to 5% by weight, more preferably from 0.5% by weight to 4.8% by weight and most preferably from 1% by weight to 4.5% by weight, calculated to the total weight of the composition.

In the case that the cosmetic composition of the present invention contains homosalate it is preferred if the total quantity of homosalate is in the range from 1% by weight to 15% by weight, more preferably from 3% by weight to 10% by weight and most preferably from 5% by weight to 8% by weight, calculated to the total weight of the composition.

In the case that the cosmetic composition of the present invention contains ethylhexyl salicylate it is preferred if the total quantity of ethylhexyl salicylate is in the range from 1% by weight to 12% by weight, more preferably from 2% by weight to 8% by weight and most preferably from 3% by weight to 6% by weight, calculated to the total weight of the composition.

In the case that the cosmetic composition of the present invention contains octocrylene it is preferred if the total quantity of octocrylene is in the range from 0.1% by weight to 10% by weight, more preferably from 1% by weight to 9% by weight and most preferably from 2% by weight to 8% by weight, calculated to the total weight of the composition.

In the case that the cosmetic composition of the present invention contains butyl methoxydibenzoylmethane it is preferred if the total quantity of butyl methoxydibenzoylmethane is in the range from 0.5% by weight to 10% by weight, more preferably from 1% by weight to 8% by weight and most preferably from 3% by weight to 5% by weight, calculated to the total weight of the composition.

Furthermore, preferred compositions according to the invention are therein characterized that the compositions do not contain ethylhexyl methoxycinnamate.

Moreover, the cosmetic compositions of the present invention are preferably free from inorganic UV-filter, especially free from zinc oxide and titanium dioxide. It is especially preferred, if the total quantity of zinc oxide and titanium dioxide in the cosmetic composition of the present invention is less than 0.1% by weight and more preferably 0% by weight, calculated to the total weight of the composition.

In general, it is the case that cosmetic compositions are preferred which are characterized in that the total quantity oil phase of the composition is in the range from 5% by weight to 40% by weight, more preferably 6% by weight to 35% by weight and most preferably 7% to 20% by weight, calculated to the total weight of the composition. According to the invention, emulsifiers do not count to the total quantity of the oil phase.

It is furthermore preferred if the cosmetic composition comprises at least one aqueous phase. As emulsifiers do not count to the oil phase of the cosmetic composition, they are counted to the total quantity of the aqueous phase.

Regarding to the cosmetic composition of the present invention it is preferred if the total quantity of water in the composition is in the range from 65% to 95% by weight, more preferably from 75% to 93% by weight and most preferably from 80% to 90% by weight, calculated to the total weight of the composition.

According to the invention, it is further preferred if the cosmetic composition contains phenoxyethanol. In the case the cosmetic composition contains phenoxyethanol the total quantity of phenoxyethanol is preferably in the range from 0.1 % by weight to 2% by weight and more preferably from 0.4% by weight to 1% by weight, calculated to the total weight of the composition.

Moreover, preferred cosmetic compositions of the present invention are therein characterized that they contain ethylhexylglycerin, whereby it is further preferred if the total quantity of ethylhexylglycerin is in the range from 0.05% to 0.5% by weight, calculated to the total weight of the composition.

Furthermore, it is preferred if the composition does not contain an alkylparabene such as methyl parabene and/or butyl parabene.

It is also preferred according to the invention if the cosmetic composition according to the invention does not contain antiperspirant active ingredients, in particular no aluminum salts such as aluminum chlorohydrate.

Moreover, it is preferred according to the present invention if the cosmetic composition contains polymers which are polymerized from a mixture containing polyols and di- or polyisocyanate in a total quantity of 0% by weight, calculated to the total weight of the composition.

Further preferred cosmetic compositions according to the invention contain glycerol in a total quantity from 1% to 10%, more preferably from 2% to 8% by weight calculated to the total weight of the composition.

In addition, preferred cosmetic compositions are characterized in that they contain ethanol in a total quantity of less than 0.5% by weight, more preferably less than 0.2% by weight and most preferably 0% by weight, calculated to the total weight of the composition.

According to the invention the cosmetic composition may preferably contain at least one compound selected from the group of limonene, linalool, citral, alpha-isomethyl ionone and geraniol.

Moreover, it is further preferred if the cosmetic composition according to the invention has a pH value in the range from 4.5 to 8.

Additionally, preferred cosmetic composition of the present invention are therein characterized that they have a viscosity in the range from 3000 mPa·s to 7000 mPa·s, whereby the viscosity is measured using a Brookfield RV viscometer, spindle RV 4, 20 rpm without Helipath, at 20°C ambient temperature and 20°C sample temperature.

Furthermore, it is advantageous according to the invention if the cosmetic composition contains one or more compounds selected from the group of alpha-lipoic acid, folic acid, phytoene, D-biotin, coenzyme Q10, alpha-glucosylrutin, carnitine, carnosine, natural and / or synthetic isoflavonoids, flavonoids, creatine, creatinine, taurine, β-alanine, tocopheryl acetate, dihydroxyacetone, glycyrrhetinic acid, 8-hexadecene-1, 16-dicarboxylic acid, glycerylglycose, (2-hydroxyethyl) urea and/or licochalcone A.

In the case the cosmetic composition comprises at least one oil phase and at least one aqueous phase it is generally preferred, if at least one oil phase is dispersed in an aqueous phase.

One type of cosmetic compositions comprising an oil phase dispersed in an aqueous phase are known as oil-in-water emulsions. The at least two phases contained in an emulsion are stabilized by emulsifiers, avoiding a phase separation.

A first preferred embodiment of the invention is characterized in that the cosmetic composition is an oil-in-water emulsion. Within this first preferred embodiment it is preferred if the cosmetic composition contains at least one emulsifier having a HLB value in the range from 8 to 18, more preferably in the range from 10 to 15. One emulsifier, which is especially preferred is glyceryl stearate citrate.

Within the first preferred embodiment of the invention, it is also further preferred if the cosmetic composition comprises at least one polymeric rheology modifier, which is preferably a carbomer. If the composition comprises at least one polymeric rheology modifier, it is preferred if the total quantity of the polymeric rheology modifier is in the range from 0.05% to 0.6% by weight, more preferred from 0.1% to 0.3% by weight and most preferred from 0.15% to 0.25% by weight, calculated to the total weight of the composition. Accordingly it is preferred if the composition comprises at least carbomer and the total quantity of the carbomer is in the range from 0.05% to 0.6% by weight, more preferred from 0.1% to 0.3% by weight and most preferred from 0.15% to 0.25% by weight, calculated to the total weight of the composition.

Another type of a two-phase cosmetic composition is known as hydrodispersion. Hydrodispersions consist of an oil phase dispersed in an external aqueous phase, whereby the stability of the multiphase system is ensured in that the aqueous phase contains a gelling agent forming a gel structure, in which the droplets of the oil phase are stably suspended. Therefore, in contrast to emulsions, hydrodispersions are stable in the absence of emulsifier.

Therefore, a second preferred embodiment of the invention is characterized in that the composition comprises at least one gel-forming polymer and the total quantity of emulsifiers is less than 2% by weight, more preferably less than 1% by weight, still more preferably less than 0.5% by weight, still more preferably less than 0.2% by weight and most preferably 0% by weight, calculated to the total weight of the composition.

One preferred type of gel-forming polymer is known under the CTFA declaration acrylates/C10-30 alkyl acrylate crosspolymer. Hence, it is preferred, if the cosmetic composition according to the second preferred embodiment of the invention contains at least one acrylates/C10-30 alkyl acrylate crosspolymer.

Hence, it is preferred if the cosmetic composition comprises at least one acrylates/C10-30 alkyl acrylate crosspolymer and the total quantity of emulsifiers is less than 2% by weight, more preferably less than 1% by weight, still more preferably less than 0.5% by weight, still more preferably less than 0.2% by weight and most preferably 0% by weight, calculated to the total weight of the composition.

Within the second embodiment of the invention it is further preferred if the composition contains as gel-forming agent at least one acrylates/C10-30 alkyl acrylate crosspolymer, whereby the total quantity of the acrylates/C10-30 alkyl acrylate crosspolymer is in the range from 0.05% to 2% by weight, more preferably 0.1% to 1% by weight and most preferably 0.2% to 0.7% by weight, calculated to the total weight of the composition.

It was surprisingly noticed, that the water and/or sweat evaporation rate is very high, if the acrylates/C10-30 alkyl acrylate crosspolymer is defined by the fact that, if the polymer is dissolved in a total quantity of 0.2% by weight in water and the mixture is adjusted to a pH of 7.5, the resulting mixture has a viscosity in the range from 6500 mPa·s to 8000 mPa·s, whereby the viscosity is measured using a Brookfield RV viscometer, spindle RV 4, 20 rpm without Helipath, at 20° C ambient temperature and 20 °C sample temperature.

According to the invention the pH-adjustment for the measurement of the viscosity of the acrylates/C10-30 alkyl acrylate crosspolymer is either done by the addition of sodium hydroxide or citric acid.

An acrylates/C10-30 alkyl acrylate crosspolymer which is according to the present invention is commercially available under the trade name Pemulen^{™} TR-1 sold by the Fa Lubrizol. Viscosity measurements which were made according to invention indicated a viscosity of 7400 mPa·s for Pemulen^{™} TR-1. The acrylates/C10-30 alkyl acrylate crosspolymer which is sold under the trade name Pemulen^{™} EZ-4U by the Fa Lubrizol has a viscosity of 4900 mPa·s, measured as specified above.

Hence, within the second preferred embodiment it is especially preferred, if the cosmetic composition contains, calculated to the total weight of the composition, 0.05% to 2% by weight, more preferably 0.1% to 1% by weight and most preferably 0.2% to 0.7% by weight of at least one acrylates/C10-30 alkyl acrylate crosspolymer, which is defined by the fact that, if the polymer is dissolved in a total quantity of 0.2% by weight in water and the mixture is adjusted to a pH of 7.5, the resulting mixture has a viscosity in the range from 6500 mPa·s to 8000 mPa·s, whereby the viscosity is measured using a Brookfield RV viscometer, spindle RV 4, 20 rpm without Helipath, at 20° C ambient temperature and 20 °C sample temperature.

It is further preferred within the second embodiment of the present invention, if the ratio by weight between the total quantity by weight of the oil a) and the acrylates/C10-30 alkyl acrylate crosspolymer is in the range from 1:1 to 100:1, more preferably 4:1 to 40:1 and most preferably 7:1 to 13:1.

Within the second embodiment of the invention it is also generally preferred, if the total quantity of any further polymeric rheology modifier, in particular polysaccharides, chemically modified polysaccharides, carbomers, sodium polyacrylate and/or further non-crosslinked polymers which are polymerized from a mixture containing acrylic acid, methacrylic acid, vinylpyrrolidone and/or vinyl acetate, is less than 0.5% by weight, more preferably less than 0.15% by weight and most preferably 0% by weight, calculated to the total weight of the composition. That means it most preferred if the cosmetic composition does not contain a rheology modifier selected from the group of polysaccharides, chemically modified polysaccharides, carbomers, sodium polyacrylate and/or further non-crosslinked polymers which are polymerized from a mixture containing acrylic acid, methacrylic acid, vinylpyrrolidone and/or vinyl acetate.

Generally, the cosmetic compositions according to the present invention can be prepared by common formulation techniques for emulsions. However, in the case the cosmetic composition according to the invention contains an UV-filter which is soluble in water it may happen that the water-soluble UV-filter affects the gel formation of the acrylates/C10-30 alkyl acrylate crosspolymer resulting in a loss of viscosity. It was surprisingly found that this problem can be addressed by adding the water-soluble UV-filter after the mixing of all other ingredients. This means that if the composition of the present invention contains at least one water soluble UV-filter and a gel forming agent, it is preferred that the water-soluble UV-filter is/are added after the formation of the gel structure.

### Examples:

The following examples should illustrate the compositions of this invention, without intending to limit the invention to these examples. The numerical values in the examples are percentages by weight, based on the total weight of the preparations.

**Table 2:**

| | Sauna 99 | Sauna 142 | Sauna 140 | Sauna 142 |
|---|---|---|---|---|
| INCl | Inv.1 | Com.1 | Inv.2 | Com. 2 |
| Diisopropyl Adipate | 5 | | 5 | |
| Paraffinum Liquidum | | 5 | | 5 |
| Butylene Glycol Dicaprylate/Dicaprate | 1 | 1 | 1 | 1 |
| Dimethicone | 0.3 | 0.3 | 0.3 | 0.3 |
| Butyrospermum Parkii Butter | 1 | 1 | 1 | 1 |
| Cetearyl Alcohol | 2 | 2 | 2 | 2 |
| Glycerin | 6 | 6 | 6 | 6 |
| Phenoxyethanol | 0.8 | 0.8 | 0.8 | 0.8 |
| Ethylhexylglycerin | 0.15 | 0.15 | 0.15 | 0.15 |
| Glyceryl Glucoside | 0.005 | 0.005 | 0.005 | 0.005 |
| Acrylates/C10-30 Alkyl Acrylate Crosspolymer (Pemulen TR-1, Lubrizol) | 0.5 | 0.5 | | |
| Acrylates/C10-30 Alkyl Acrylate Crosspolymer (Pemulen EZ4U; Lubrizol ) | | | 0.5 | 0.5 |
| Sodium Hydroxide | q.s. | q.s. | q.s. | q.s. |
| Parfum | q.s. | q.s. | q.s. | q.s. |
| Aqua | ad 100 | ad 100 | ad 100 | ad 100 |
| | | | | |
| Av. evaporation factor | 2.2 | 4.1 | 3.05 | 4.64 |

The sweat evaporation after application of the compositions listed in table 2 (Inv.1, Inv.2, Com. 1 and Com. 2) was investigated in the following study:
1. 50µl of each of the cosmetic compositions presented in table 2 were applied to a marked section of 20 cm² on the forearm (one per composition) of a volunteer taking part in the study. In addition one marked section of 20 cm² remained untreated as reference.
2. It was waited for 10 minutes to let the compositions absorb.
3. Afterwards the volunteer spent 10 minutes in a sauna room at a temperature of 50°C and a humidity 55%.
4. After leaving the sauna the time was measured until the sweat on the marked sections had evaporated. For the measurement the volunteers indicated when the marked section felt dry.
5. To standardize the measurement, the determined drying time for the individual section treated with a cosmetic composition was divided by the time it took for the non-treated section to dry. Hence, the lower the determined factor (called evaporation factor) the higher is the evaporation rate. The average value of the determined evaporation factors for each composition is listed in table 2.

In total 11 volunteers took part in the study, whereby each composition was evaluate once by each volunteer.

By looking at the evaporation factor for the different formulations it can be established that in both comparison experiments (Inv.1 vs. Com.1 and Inv.2 vs Com.2) the evaporation rate for the formulations according to the invention was significantly higher than for the comparison examples. This is indicated by the fact that the evaporation factor is lower for the inventive compositions. None of the inventive compositions left white residues behind on the skin after drying.

It was further investigated if the compositions disclosed in table 2 had an antiperspirant effect. The experiments undertaken indicated that none of the compositions had an antiperspirant effect.

The following examples shall further exemplify the present invention.

| INCl | Ex. 1 | Ex. 2 | Ex. 3 | Ex. 4 | |
|---|---|---|---|---|---|
| Glyceryl Glucoside | 0.005 | | 0.005 | 0.005 | |
| Ethylhexylglycerin | 0.15 | | 0.15 | 0.15 | |
| Diisopropyl Adipate | 8 | 3 | 5 | 6.5 | |
| Butylene Glycol Dicaprylate/Dicaprate | 1 | | 1 | 1 | |
| Dimethicone | 0.3 | 0.3 | 0.3 | 0.3 | |
| Glycerin | 6 | | 6 | 6 | |
| Sodium Hydroxide | qs. | qs. | qs. | qs. | |
| Phenoxyethanol | 0.8 | 0.8 | 0.8 | 0.8 | |
| Acrylates/C10-30 Alkyl Acrylate Crosspolymer (Pemulen EZ4U) | 0.3 | 0.5 | 0.5 | | |
| Acrylates/C10-30 Alkyl Acrylate Crosspolymer (Pemulen TR-1) | | | | 0.5 | |
| Carbomer | | | 0.3 | 0.3 | |
| Homosalate | | 8 | | | |
| Octocrylene | | 2 | | | |
| Ethylhexyl Salicylate | | 4.5 | | | |
| Bis-Ethylhexyloxyphenol Methoxyphenyl Triazine | | 1 | | | |
| Butyl Methoxydibenzoylmethane | | 3.5 | | | |
| Aqua | ad 100 | ad 100 | ad 100 | ad 100 | |
| | | | | | |

| INCl | Ex. 5 | Ex. 6 | Ex. 7 | Ex.8 | Ex.9 |
|---|---|---|---|---|---|
| Glyceryl Glucoside | 0.005 | 0.005 | 0.005 | 0.005 | 0.005 |
| Ethylhexylglycerin | 0.15 | 0.15 | 0.15 | 0.15 | 0.15 |
| Diisopropyl Adipate | 6.5 | 3 | 4 | 5 | 5 |
| Butylene Glycol Dicaprylate/Dicaprate | 1 | 1 | 1 | 1 | 1 |
| Dimethicone | 0.3 | 0.3 | 0.3 | 0.3 | 0.3 |
| Glycerin | 6 | 6 | 6 | 6 | 6 |
| Butyrospermum Parkii Butter | | | | 1 | 1 |
| Glyceryl Stearate Citrate | | | | 2 | 2 |
| Sodium Hydroxide | q.s. | q.s. | q.s. | q.s. | q.s. |
| Phenoxyethanol | 0.8 | 0.8 | 0.8 | 0.8 | 0.8 |
| Acrylates/C10-30 Alkyl Acrylate Crosspolymer (Pemulen EZ4U) | 0.5 | | 0.2 | | |
| Acrylates/C10-30 Alkyl Acrylate Crosspolymer (Pemulen TR-1) | | 0.2 | 0.3 | | |
| Carbomer (Carbopol 980; Lubrizol) | | | | 0.4 | 0.2 |
| Ceteraryl Alcohol | 1 | | | 2 | 2 |
| Aqua | ad 100 | ad 100 | ad 100 | ad 100 | ad 100 |
| | | | | | |

| INCl | Ex. 10 | Ex. 11 | Ex. 12 | Ex. 13 | |
|---|---|---|---|---|---|
| Glyceryl Glucoside | 0.005 | | 0.005 | 0.005 | |
| Ethylhexylglycerin | 0.15 | 0.2 | 0.15 | 0.15 | |
| Lauryl Lactate | 1 | 4 | | | |
| C-12-15 Alkyl Lactate | | | 3.5 | | |
| Diisopropyl Adipate | 5 | | | | |
| Diisopropyl Sebacate | | 0.1 | | 6.5 | |
| Butylene Glycol Dicaprylate/Dicaprate | 1 | 0.5 | 1 | 1 | |
| Dimethicone | 0.3 | 0.3 | 0.3 | 0.3 | |
| Glycerin | 6 | | 6 | 6 | |
| Sodium Hydroxide | qs. | qs. | qs. | qs. | |
| Phenoxyethanol | 0.8 | 0.8 | 0.8 | 0.8 | |
| Acrylates/C10-30 Alkyl Acrylate Crosspolymer (Pemulen EZ4U) | 0.35 | 0.45 | 0.25 | 0.05 | |
| Acrylates/C10-30 Alkyl Acrylate Crosspolymer (Pemulen TR-1) | | | 0.25 | 0.45 | |
| Carbomer | 0.1 | | 0.3 | 0.3 | |
| Homosalate | 3 | 8 | | | |
| Octocrylene | 1.2 | 2 | | 2 | |
| Ethylhexyl Salicylate | 3 | 4.5 | 3 | | |
| Bis-Ethylhexyloxyphenol Methoxyphenyl Triazine | 0.8 | 1 | 2 | | |
| Butyl Methoxydibenzoylmethane | 2.5 | 3.5 | | | |
| Aqua | ad 100 | ad 100 | ad 100 | ad 100 | |
| | | | | | |

| INCl | Ex. 14 | Ex. 15 | Ex. 16 | Ex.17 | Ex.18 |
|---|---|---|---|---|---|
| Glyceryl Glucoside | 0.005 | 0.005 | 0.005 | 0.005 | 0.005 |
| Ethylhexylglycerin | 0.15 | 0.15 | 0.15 | 0.15 | 0.15 |
| Lauryl Lactate | 5.5 | | | 5 | |
| C-12-15 Alkyl Lactate | | 3 | | | 5 |
| Diisopropyl Adipate | 1 | 2 | | | |
| Diisopropyl Sebacate | | | 4 | | |
| Butylene Glycol Dicaprylate/Dicaprate | 1 | 1 | 1 | 1 | 1 |
| Dimethicone | 0.3 | 0.3 | 0.3 | 0.3 | 0.3 |
| Glycerin | 6 | 6 | 6 | 6 | 6 |
| Butyrospermum Parkii Butter | | | | 1 | 1 |
| Glyceryl Stearate Citrate | | | | 2 | 2 |
| Sodium Hydroxide | q.s. | q.s. | q.s. | q.s. | q.s. |
| Phenoxyethanol | 0.8 | 0.8 | 0.8 | 0.8 | 0.8 |
| Acrylates/C10-30 Alkyl Acrylate Crosspolymer (Pemulen EZ4U) | 0.5 | | 0.2 | | |
| Acrylates/C10-30 Alkyl Acrylate Crosspolymer (Pemulen TR-1) | | 0.2 | 0.3 | | |
| Carbomer (Carbopol 980; Lubrizol | | | | 0.4 | 0.2 |
| Ceteraryl Alcohol | 1 | | | 2 | 2 |
| Aqua | ad 100 | ad 100 | ad 100 | ad 100 | ad 100 |

## Claims

1. The use of an oil a) having a dielectric constant of at least 4.0 in a cosmetic composition to increase after application of the composition to the human skin the evaporation rate of water and/or sweat from the skin;

2. The use according to claims 1 **characterized in that** the dielectric constant of the oil a) is at least 4.5, more preferably at least 5.0 and most preferably at least 5.2.

3. The use according to claim 1 or 2 **characterized in that** the dielectric constant of the oil a) is less than 8.0, more preferred less than 7.0 and most preferred less than 6.1.

4. The use according to any of the preceding claims **characterized in that** the oil a) contains at least one ester group in its molecular structure.

5. The use according to any of the preceding claims **characterized in that** the oil a) is not an organic UV-filter.

6. The use according to any of the preceding claims **characterized in that** the oil a) is chosen from the group of isodecyl malate, C12-15 alkyl lactate, lauryl lactate, dibutyl adipate, diisopropyl adipate, diethylhexyl adipate and diisopropyl sebacate.

7. The use according to any of the preceding claims **characterized in that** the oil a) is chosen from the group of isodecyl malate, C12-15 alkyl lactate, lauryl lactate and diisopropyl adipate.

8. The use according to any of the preceding claims **characterized in that** the oil a) is diisopropyl adipate.

9. The use according to any of the preceding claims **characterized in that** the total quantity of the oil a) in the cosmetic composition is in the range from 1% to 20% by weight, more preferably from 2% to 10% by weight and most preferably from 3% to 7% by weight, calculated to the total weight of the composition.

10. The use according to any of the preceding claims **characterized in that** the cosmetic composition contains at least one silicone oil in a total quantity of 0.05% to 2% by weight, more preferably 0.1% to 2% by weight and most preferably 0.2% to 0.5% by weight, calculated to the total weight of the composition.

11. The use according to any of the preceding claims **characterized in that** the cosmetic composition contains all non-silicone oils, which have a dielectric constant of less than 4.0 at 25°C and which are not an organic UV-filter, in a total quantity of less than 1.5% by weight, more preferably less than 0.8% by weight, more preferably less than 0.5% by weight, more preferably less than 0.1% by weight and most preferably 0% by weight, calculated to the total weight of the composition.

12. The use according to any of the preceding claims **characterized in that** the cosmetic composition contains C12-15 alkyl benzoate, mineral oil, caprylic/capric triglyceride and/or ethylhexyl palmitate in a total quantity of less than 0.2% by weight, more preferably less than 0.1% by weight and most preferably 0% by weight, calculated to the total weight of the composition.

13. The use according to any of the preceding claims **characterized in that** the cosmetic composition contains cetyl alcohol and/or stearyl alcohol.

14. The use according to any of the preceding claims **characterized in that** the cosmetic composition contains at least one organic UV-filter, which is preferably selected from the group of camphor benzalkonium methosulfate, homosalate, benzophenone-3, phenylbenzimidazole sulfonic acid, terephthalylidene dicamphor sulfonic Acid, butyl methoxydibenzoylmethane, benzylidene camphor sulfonic acid, octocrylene, polyacrylamidomethyl benzylidene camphor, PEG-PABA, isoamyl P-methoxycinnamate, ethylhexyl triazone, drometrizole trisiloxane, diethylhexyl butamido triazone, 4-methylbenzylidene camphor, ethylhexyl salicylate, ethylhexyl dimethyl PABA, benzohenone-4, benzophenone-5, methylene bis-benzotriazolyl tetramethylbutylphenol, disodium phenyl dibenzimidazole tetrasulfonate, bis-ethylhexyloxyphenol methoxyphenyl triazine, polysilicone-15, diethylamino hydroxybenzoyl hexyl benzoate and tris-biphenyl triazine.

15. The use according to any of the preceding claims **characterized in that** the total quantity of the organic UV-filters contained in the cosmetic composition is in the range from 1% by weight to 35% by weight, more preferably 8% by weight to 30% by weight and most preferably 10% by weight to 28% by weight, calculated to the total weight of the composition.

16. The use according to any of the preceding claims **characterized in that** the cosmetic composition comprises at least one oil phase and at least one aqueous phase, whereby it is preferred, if at least one oil phase is dispersed in an aqueous phase.

17. The use according to any of the preceding claims **characterized in that** the total quantity of water in the composition is in the range from 65% to 95% by weight, more preferably from 75% to 93% by weight and most preferably from 80% to 90% by weight, calculated to the total weight of the composition.

18. The use according to any of the preceding claims **characterized in that** the cosmetic composition is an oil-in-water emulsion and the cosmetic composition contains at least one emulsifier having a HLB value in the range from 8 to 18, more preferably in the range from 10 to 15.

19. The use according to any of the preceding claims **characterized in that** the cosmetic composition comprises at least carbomer and the total quantity of the carbomer is in the range from 0.05% to 0.6% by weight, more preferred from 0.1% to 0.3% by weight and most preferred from 0.15% to 0.25% by weight, calculated to the total weight of the composition.

20. The use according to any of the claims 1 to 17 **characterized in that** the cosmetic composition comprises at least one acrylates/C10-30 alkyl acrylate crosspolymer and the total quantity of emulsifiers is less than 2% by weight, more preferably less than 1% by weight, still more preferably less than 0.5% by weight, still more preferably less than 0.2% by weight and most preferably 0% by weight, calculated to the total weight of the composition.

21. The use according to claim 20 **characterized in that** the total quantity of the acrylates/C10-30 alkyl acrylate crosspolymer is in the range from 0.05% to 2% by weight, more preferably 0.1% to 1% by weight and most preferably 0.2% to 0.7% by weight, calculated to the total weight of the composition.

22. The use according to the claims 20 or 21 **characterized in that** the acrylates/C10-30 alkyl acrylate crosspolymer is defined by the fact that, if the polymer is dissolved in a total quantity of 0.2% by weight in water and the mixture is adjusted to a pH of 7.5, the resulting mixture has a viscosity in the range from 6500 mPa·s to 8000 mPa·s, whereby the viscosity is measured using a Brookfield RV viscometer, spindle RV 4, 20 rpm without Helipath, at 20° C ambient temperature and 20 °C sample temperature.

23. The use according to any of the claims 20 to 22 **characterized in that** the ratio by weight between the total quantity by weight of the oil a) and the acrylates/C10-30 alkyl acrylate crosspolymer is in the range from 1:1 to 100:1, more preferably 4:1 to 40:1 and most preferably 7:1 to 13:1.

## Patentansprüche

1. Verwendung eines Öls a) mit einer Dielektrizitätskonstante von mindestens 4,0 in einer kosmetischen Zusammensetzung, um nach Auftragung der Zusammensetzung auf die menschliche Haut die Verdampfungsrate von Wasser und/oder Schweiß von der Haut zu erhöhen.

2. Verwendung nach Anspruch 1, **dadurch gekennzeichnet, dass** die Dielektrizitätskonstante des Öls a) mindestens 4,5, bevorzugter mindestens 5,0 und am meisten bevorzugt mindestens 5,2 beträgt.

3. Verwendung nach Anspruch 1 oder 2, **dadurch gekennzeichnet, dass** die Dielektrizitätskonstante des Öls a) weniger als 8,0, bevorzugter weniger als 7,0 und am meisten bevorzugt weniger als 6,1 beträgt.

4. Verwendung nach einem der vorhergehenden Ansprüche, **dadurch gekennzeichnet, dass** das Öl a) mindestens eine Estergruppe in seiner Molekülstruktur enthält.

5. Verwendung nach einem der vorhergehenden Ansprüche, **dadurch gekennzeichnet, dass** das Öl a) kein organischer UV-Filter ist.

6. Verwendung nach einem der vorhergehenden Ansprüche, **dadurch gekennzeichnet, dass** das Öl a) ausgewählt ist aus der Gruppe von Isodecylmalat, C₁₂₋₁₅-Alkyllactat, Lauryllactat, Dibutyladipat, Diisopropyladipat, Diethylhexyladipat und Diisopropylsebacat.

7. Verwendung nach einem der vorhergehenden Ansprüche, **dadurch gekennzeichnet, dass** das Öl a) ausgewählt ist aus der Gruppe von Isodecylmalat, C₁₂₋₁₅-Alkyllactat, Lauryllactat und Diisopropyladipat.

8. Verwendung nach einem der vorhergehenden Ansprüche, **dadurch gekennzeichnet, dass** das Öl a) Diisopropyladipat ist.

9. Verwendung nach einem der vorhergehenden Ansprüche, **dadurch gekennzeichnet, dass** die Gesamtmenge des Öls a) in der kosmetischen Zusammensetzung im Bereich von 1 Gew.% bis 20 Gew.%, bevorzugter 2 Gew.% bis 10 Gew.% und am meisten bevorzugt 3 Gew.% bis 7 Gew.% liegt, berechnet bezogen auf das Gesamtgewicht der Zusammensetzung.

10. Verwendung nach einem der vorhergehenden Ansprüche, **dadurch gekennzeichnet, dass** die kosmetische Zusammensetzung mindestens ein Silikonöl in einer Gesamtmenge von 0,05 Gew.% bis 2 Gew.%, bevorzugter 0,1 Gew.% bis 2 Gew.% und am meisten bevorzugt 0,2 Gew.% bis 0,5 Gew.% enthält, berechnet bezogen auf das Gesamtgewicht der Zusammensetzung.

11. Verwendung nach einem der vorhergehenden Ansprüche, **dadurch gekennzeichnet, dass** die kosmetische Zusammensetzung alle Nicht-Silikonöle, die eine Dielektrizitätskonstante von weniger als 4,0 bei 25 °C haben und die kein organischer UV-Filter sind, in einer Gesamtmenge von weniger als 1,5 Gew.%, bevorzugter weniger als 0,8 Gew.%, bevorzugter weniger als 0,5 Gew.%, bevorzugter weniger als 0,1 Gew.% und am meisten bevorzugt 0 Gew.% enthält, berechnet bezogen auf das Gesamtgewicht der Zusammensetzung.

12. Verwendung nach einem der vorhergehenden Ansprüche, **dadurch gekennzeichnet, dass** die kosmetische Zusammensetzung C₁₂₋₁₅-Alkylbenzoat, Mineralöl, Capryl-/Caprintriglycerid und/oder Ethylhexylpalmitat in einer Gesamtmenge von weniger als 0,2 Gew.%, bevorzugter weniger als 0,1 Gew.% und am meisten bevorzugt 0 Gew.% enthält, berechnet bezogen auf das Gesamtgewicht der Zusammensetzung.

13. Verwendung nach einem der vorhergehenden Ansprüche, **dadurch gekennzeichnet, dass** die kosmetische Zusammensetzung Cetylalkohol und/oder Stearylalkohol enthält.

14. Verwendung nach einem der vorhergehenden Ansprüche, **dadurch gekennzeichnet, dass** die kosmetische Zusammensetzung mindestens einen organischen UV-Filter enthält, der vorzugsweise ausgewählt ist aus der Gruppe von Kampherbenzalkoniummethosulfat, Homosalat, Benzophenon-3, Phenylbenzimidazolsulfonsäure, Terephthalylidendikamphersulfonsäure, Butylmethoxydibenzoylmethan, Benzylidenkamphersulfonsäure, Octocrylen, Polyacrylamidomethylbenzylidenkampher, PEG-PABA, Isoamyl-P-methoxycinnamat, Ethylhexyltriazon, Drometrizoltrisiloxan, Diethylhexylbutamidotriazon, 4-Methylbenzylidenkampher, Ethylhexylsalicylat, Ethylhexyldimethyl-PABA, Benzophenon-4, Benzophenon-5, Methylenbisbenzotriazolyltetramethylbutylphenol, Dinatriumphenyldibenzimidazoltetrasulfonat, Bisethylhexyloxyphenolmethoxyphenyltriazin, Polysilikon-15, Diethylaminohydroxybenzoylhexylbenzoat und Trisbiphenyltriazin.

15. Verwendung nach einem der vorhergehenden Ansprüche, **dadurch gekennzeichnet, dass** die Gesamtmenge der organischen UV-Filter, die in der kosmetischen Zusammensetzung enthalten sind, im Bereich von 1 Gew.% bis 35 Gew.%, bevorzugter 8 Gew.% bis 30 Gew.% und am meisten bevorzugt 10 Gew.% bis 28 Gew.% liegt, berechnet bezogen auf das Gesamtgewicht der Zusammensetzung.

16. Verwendung nach einem der vorhergehenden Ansprüche, **dadurch gekennzeichnet, dass** die kosmetische Zusammensetzung mindestens eine Ölphase und mindestens eine wässrige Phase umfasst, wobei bevorzugt ist, wenn mindestens eine Ölphase in einer wässrigen Phase dispergiert ist.

17. Verwendung nach einem der vorhergehenden Ansprüche, **dadurch gekennzeichnet, dass** die Gesamtmenge des Wassers in der Zusammensetzung im Bereich von 65 Gew.% bis 95 Gew.%, bevorzugter 75 Gew.% bis 93 Gew.% und am meisten bevorzugt 80 Gew.% bis 90 Gew.% liegt, berechnet bezogen auf das Gesamtgewicht der Zusammensetzung.

18. Verwendung nach einem der vorhergehenden Ansprüche, **dadurch gekennzeichnet, dass** die kosmetische Zusammensetzung eine Öl-in-Wasser-Emulsion ist und die kosmetische Zusammensetzung mindestens einen Emulgator mit einem HLB-Wert im Bereich von 8 bis 18, bevorzugter im Bereich von 10 bis 15 enthält.

19. Verwendung nach einem der vorhergehenden Ansprüche, **dadurch gekennzeichnet, dass** die kosmetische Zusammensetzung mindestens Carbomer umfasst und die Gesamtmenge des Carbomers im Bereich von 0,05 Gew.% bis 0,6 Gew.%, bevorzugter 0,1 Gew.% bis 0,3 Gew.% und am meisten bevorzugt 0,15 Gew.% bis 0,25 Gew.% liegt, berechnet bezogen auf das Gesamtgewicht der Zusammensetzung.

20. Verwendung nach einem der Ansprüche 1 bis 17, **dadurch gekennzeichnet, dass** die kosmetische Zusammensetzung mindestens ein Acrylate/C₁₀₋₃₀-Alkylacrylat-Kreuzpolymer umfasst und die Gesamtmenge an Emulgatoren weniger als 2 Gew.%, bevorzugter weniger als 1 Gew.%, noch bevorzugter weniger als 0,5 Gew.%, noch bevorzugter weniger als 0,2 Gew.% und am meisten bevorzugt 0 Gew.% beträgt, berechnet bezogen auf das Gesamtgewicht der Zusammensetzung.

21. Verwendung nach Anspruch 20, **dadurch gekennzeichnet, dass** die Gesamtmenge des Acrylate/C₁₀₋₃₀-Alkylacrylat-Kreuzpolymers im Bereich von 0,05 Gew.% bis 2 Gew.%, bevorzugter 0,1 Gew.% bis 1 Gew.% und am meisten bevorzugt 0,2 Gew.% bis 0,7 Gew.% liegt, berechnet bezogen auf das Gesamtgewicht der Zusammensetzung.

22. Verwendung nach den Ansprüchen 20 oder 21, **dadurch gekennzeichnet, dass** das Acrylate/C₁₀₋₃₀-Alkylacrylat-Kreuzpolymer durch die Tatsache definiert ist, dass, wenn das Polymer in einer Gesamtmenge von 0,2 Gew.% in Wasser gelöst wird und die Mischung auf einen pH-Wert von 7,5 eingestellt wird, die resultierende Mischung eine Viskosität im Bereich von 6500 mPa·s bis 8000 mPa·s hat, wobei die Viskosität unter Verwendung eines Brookfield RV-Viskosimeters, Spindel RV 4, 20 UpM ohne Helipath bei 20 °C Umgebungstemperatur und 20 °C Probentemperatur gemessen wird.

23. Verwendung nach einem der Ansprüche 20 bis 22, **dadurch gekennzeichnet, dass** das Gewichtsverhältnis zwischen der gewichtsbezogenen Gesamtmenge des Öls a) und des Acrylate/C₁₀₋₃₀-Alkylacrylat-Kreuzpoly-mers im Bereich von 1:1 bis 100:1, bevorzugter 4:1 bis 40:1 und am meisten bevorzugt 7:1 bis 13:1 liegt.

## Revendications

1. Utilisation d'une huile a) possédant une constante diélectrique d'au moins 4,0 dans une composition cosmétique pour augmenter la vitesse d'évaporation de l'eau et/ou de la sueur de la peau après application de la composition sur la peau humaine.

2. Utilisation selon la revendication 1, **caractérisée en ce que** la constante diélectrique de l'huile a) est d'au moins 4,5, plus préférablement d'au moins 5,0 et le plus préférablement d'au moins 5,2.

3. Utilisation selon la revendication 1 ou 2, **caractérisée en ce que** la constante diélectrique de l'huile a) est inférieure à 8,0, de manière plus préférée inférieure à 7,0 et de la manière la plus préférée inférieure à 6,1.

4. Utilisation selon l'une quelconque des revendications précédentes, **caractérisée en ce que** l'huile a) contient au moins un groupe ester dans sa structure moléculaire.

5. Utilisation selon l'une quelconque des revendications précédentes, **caractérisée en ce que** l'huile a) n'est pas un filtre UV organique.

6. Utilisation selon l'une quelconque des revendications précédentes **caractérisée, en ce que** l'huile a) est choisie dans le groupe composé du malate d'isodécyle, d'un lactate d'alkyle en C12 à C15, du lactate de lauryle, de l'adipate de dibutyle, de l'adipate de diisopropyle, de l'adipate de diéthylhexyle et du sébaçate de diisopropyle.

7. Utilisation selon l'une quelconque des revendications précédentes, **caractérisée en ce que** l'huile a) est choisie dans le groupe composé du malate d'isodécyle, d'un lactate d'alkyle en C12 à C15, du lactate de lauryle et de l'adipate de diisopropyle.

8. Utilisation selon l'une quelconque des revendications précédentes, **caractérisée en ce que** l'huile a) est l'adipate de diisopropyle.

9. Utilisation selon l'une quelconque des revendications précédentes, **caractérisée en ce que** la quantité totale de l'huile a) dans la composition cosmétique est dans la plage de 1 % à 20 % en poids, plus préférablement de 2 % à 10 % en poids et le plus préférablement de 3 % à 7 % en poids, calculée sur le poids total de la composition.

10. Utilisation selon l'une quelconque des revendications précédentes, **caractérisée en ce que** la composition cosmétique contient au moins une huile de silicone en une quantité totale de 0,05 % à 2 % en poids, plus préférablement de 0,1 % à 2 % en poids et le plus préférablement de 0,2 % à 0,5 % en poids, calculée sur le poids total de la composition.

11. Utilisation selon l'une quelconque des revendications précédentes, **caractérisée en ce que** la composition cosmétique contient toutes huiles non de silicone, qui possèdent une constante diélectrique inférieure à 4,0 à 25 °C et qui ne sont pas un filtre UV organique, en une quantité totale inférieure 1,5 % en poids, plus préférablement inférieure à 0,8 % en poids, plus préférablement inférieure à 0,5 % en poids, plus préférablement inférieure à 0,1 % en poids, et le plus préférablement de 0 % en poids, calculée sur le poids total de la composition.

12. Utilisation selon l'une quelconque des revendications précédentes, **caractérisée en ce que** la composition cosmétique contient un benzoate d'alkyle en C12 à C15, une huile minérale, un triglycéride caprylique/caprique et/ou du palmitate d'éthylhexyle en une quantité totale inférieure à 0,2 % en poids, plus préférablement inférieure à 0,1 % en poids et le plus préférablement de 0 % en poids, calculée sur le poids total de la composition.

13. Utilisation selon l'une quelconque des revendications précédentes, **caractérisée en ce que** la composition cosmétique contient de l'alcool cétylique et/ou de l'alcool stéarylique.

14. Utilisation selon l'une quelconque des revendications précédentes, **caractérisée en ce que** la composition cosmétique contient au moins un filtre UV organique, qui est préférablement choisi dans le groupe composé par le méthosulfate camphre de benzalkonium, l'homosalate, la benzophénone-3, l'acide phénylbenzimidazole sulfonique, l'acide téréphtalidène dicamphre sulfonique, le méthoxydibenzoylméthane de butyle, l'acide benzylidène camphre sulfonique, l'octocrylène, le polyacrylamidométhyl-benzylidène camphre, le PEG-PABA, le p-méthoxycinnamate d'isoamyle, l'éthylhexyl-triazone, le drométrizole trisiloxane, la diéthylhexyl-butamido triazone, le 4-méthylbenzylidène camphre, le salicylate d'éthylhexyle, l'éthylhexyl-diméthyl-PABA, la benzophénone-4, la benzophénone-5, le méthylène bis-benzotriazolyl tétraméthylbutylphénol, le phényle dibenzimidazole tétrasulfonate disodique, la bis-éthylhexyloxyphénolméthoxyphényl-triazine, la polysilicone-15, le diéthylaminohydroxybenzoylbenzoate d'hexyle et la tris-biphényl-triazine.

15. Utilisation selon l'une quelconque des revendications précédentes, **caractérisée en ce que** la quantité totale des filtres UV organiques contenus dans la composition cosmétique est dans la plage de 1 % en poids à 35 % en poids, plus préférablement de 8 % en poids à 30 % en poids et le plus préférablement de 10 % en poids à 28 % en poids, calculée sur le poids total de la composition.

16. Utilisation selon l'une quelconque des revendications précédentes, **caractérisée en ce que** la composition cosmétique comprend au moins une phase huileuse et au moins une phase aqueuse, moyennant quoi il est préféré qu'au moins une phase huileuse soit dispersée dans une phase aqueuse.

17. Utilisation selon l'une quelconque des revendications précédentes, **caractérisée en ce que** la quantité totale d'eau dans la composition est dans la plage de 65 % à 95 % en poids, plus préférablement de 75 % à 93 % en poids et le plus préférablement de 80 % à 90 % en poids, calculée sur le poids total de la composition.

18. Utilisation selon l'une quelconque des revendications précédentes, **caractérisée en ce que** la composition cosmétique est une émulsion huile-dans-eau et la composition cosmétique contient au moins un émulsifiant possédant une valeur HLB dans la plage de 8 à 18, plus préférablement dans la plage de 10 à 15.

19. Utilisation selon l'une quelconque des revendications précédentes, **caractérisée en ce que** la composition cosmétique comprend au moins un carbomère et la quantité totale du carbomère est dans la plage de 0,05 % à 0,6 % en poids, de manière plus préférée de 0,1 % à 0,3 % en poids et de la manière la plus préférée de 0,15 % à 0,25 % en poids, calculée sur le poids total de la composition.

20. Utilisation selon l'une quelconque des revendications 1 à 17, **caractérisée en ce que** la composition cosmétique comprend au moins un polymère croisé d'acrylates/acrylate d'alkyle en C10 à C30 et la quantité totale d'émulsifiants est inférieure à 2 % en poids, plus préférablement inférieure à 1 % en poids, encore plus préférablement inférieure à 0,5 % en poids, encore plus préférablement inférieure à 0,2 % en poids et le plus préférablement de 0 % en poids, calculée sur le poids total de la composition.

21. Utilisation selon la revendication 20, **caractérisée en ce que** la quantité totale du polymère croisé d'acrylates/acrylate d'alkyle en C10 à C30 est dans la plage de 0,05 % à 2 % en poids, plus préférablement de 0,1 % à 1 % en poids et le plus préférablement de 0,2 % à 0,7 % en poids, calculée sur le poids total de la composition.

22. Utilisation selon la revendication 20 ou 21, **caractérisée en ce que** le polymère croisé d'acrylates/acrylate d'alkyle en C10 à C30 est défini par le fait que si le polymère est dissous dans une quantité totale de 0,2 % en poids dans de l'eau et que le mélange est ajusté à un pH de 7,5, le mélange résultant possède une viscosité dans la plage de 6 500 mPa·s à 8 000 mPa·s, moyennant quoi la viscosité est mesurée à l'aide d'un viscosimètre Brookfield RV, broche RV 4, à 20 tpm sans Hélipath, à une température ambiante de 20 °C et une température d'échantillon de 20 °C.

23. Utilisation selon l'une quelconque des revendications 20 à 22, **caractérisée en ce que** le rapport en poids de la quantité totale en poids de l'huile a) et du polymère croisé d'acrylates/acrylate d'alkyle en C10 à C30 est dans la plage de 1 : 1 à 100 : 1, plus préférablement de 4 : 1 à 40 : 1 et le plus préférablement de 7 : 1 à 13 : 1.
